# EUROPEAN PATENT SPECIFICATION

(11) **EP 2 500 064 B1**
(45) Date of publication and mention of the grant of the patent: **08.08.2018**
(21) Application number: 12159226.5
(22) Date of filing: 13.03.2012
(51) Int. Cl.: A62C 3/06, G01N 33/00

(54) **Method and system for locating and containing submerged fires in aerated and closed chambers for composting and bio-drying of waste and storage of solid flammable materials**
Methode und Vorrichtung zum lokalisieren und eindämmen von unter der Oberfläche liegenden Bränden in belüfteten und geschlossenen Kammern zur Kompostierung und Bio-Trocknung von Müll und Lagerung von festen brennbaren Materialien
Méthode et système pour localiser et contenir les incendies submergés dans des chambres aérées et fermées pour le compostage et le bio-séchage de déchets et le stockage de matériaux solides inflammables

(30) Priority: 15.03.2011 IT MI20110408
(43) Date of publication of application: 19.09.2012
(73) Proprietor: A2A Ambiente S.P.A., 25124 Brescia (BS) (IT)
(72) Inventor: Donati, Gianni, 27010 Giussago (PV) (IT); Rossi, Valter, 27010 Giussago (PV) (IT)
(74) Representative: Porta, Checcacci & Associati S.p.A

(56) References cited:
- EP-A2- 1 258 280
- EP-A2- 1 386 675
- EP-A2- 1 466 880
- GB-A- 2 277 625

## Description

### Field of application

The present invention relates to systems for the stabilisation or composting of digestible waste and for the storage of the materials produced in this way and more generally of solid flammable materials.

More particularly the present invention relates to a system and a method for the containing of fires generated in the mass by phenomena of self-combustion or more simply by extraneous materials able to trigger incipient fires.

Some possible applications of the present invention are constituted by the disposal of waste containing a digestible fraction such as municipal solid waste (MSW), by the treatment of the residual wet fraction of the selective collection of the MSW and by the drying, stabilisation and storage of biomasses.

### State of the art

Municipal solid waste is normally subject to selective collection and gives rise to various recoverable fractions such as paper, plastic and wet waste which normally constitute approximately 30% of the original waste.

The remaining 70% after selective collection comprises an easily digestible fraction composed of wet organic residues, a non-combustible inorganic fraction made up of glass, residues from demolitions and metals, and a combustible fraction comprising packing material and material of a plastic, wood, board and paper nature. This residue is used in special incinerators to produce energy as is or appropriately treated to increase the heating value and to reduce the environmental impact.

Moreover biomasses can be advantageously obtained from land subtracted from intensive farming use and set aside, which biomasses can be used for the generation of energy.

The wet organic waste of the selective collection, like some agricultural biomasses, can be used for the production of compost by means of aerobic digestion for use in agriculture.

The residue after selective collection can be selected further or treated by means of aerobic digestion to produce a stable bio-dried material, odourless and with higher heating value.

All these materials and the products obtained from them are processed and stored in closed spaces due to process needs and to reduce the environmental impact.

The presence in the waste of materials able to set off fires such as canisters of combustible gases, the existence in the closed area of the system of machinery such as grinding mills capable of overheating during the operation pieces of metal or more commonly the triggering of self-combustion in the heap due to overheating of the dry product are potential sources for setting off incipient fires.

Methods are known for the preparation of fuels derived from waste using, for the reduction of the quantity of water present, the heat generated by the aerobic digestion of the digestible fraction present of the same waste.

In the European patent EP-A-706839 in the name of the same Applicant a method is described for the recovery of energy from municipal solid waste by means of the preparation of non-conventional fuel, comprising the reception of the waste in the closed space and the phases of rough shredding of the waste, of accumulation of the same on a porous bed, of forced digestion with temperatures up to 65 - 70°C until the waste is dried, performed by means of the passage of air aspirated through the waste itself, of removal of the odours from the output air by means of bio-filters, of refining by means of screening, of removal of the metals and of grinding of the remaining fraction until obtaining a maximum final diameter lower than one centimetre.

The waste is placed in the digestion chamber in layers also of considerable thickness, traversed by a high flow of air. After a period of induction, the oxygen contained in the air causes forced digestion of the aerobic type with production of heat and carbon dioxide.

In the Italian patent IT-A-1283805 a method is described for the recovery of energy from municipal solid waste comprising a phase of initial shredding, to homogenise and limit the formation of local pockets of anaerobic digestion, and a phase of forced digestion until drying of the waste. The flow of air removes water and the digestion stops when the remaining water is not sufficient for supporting it.

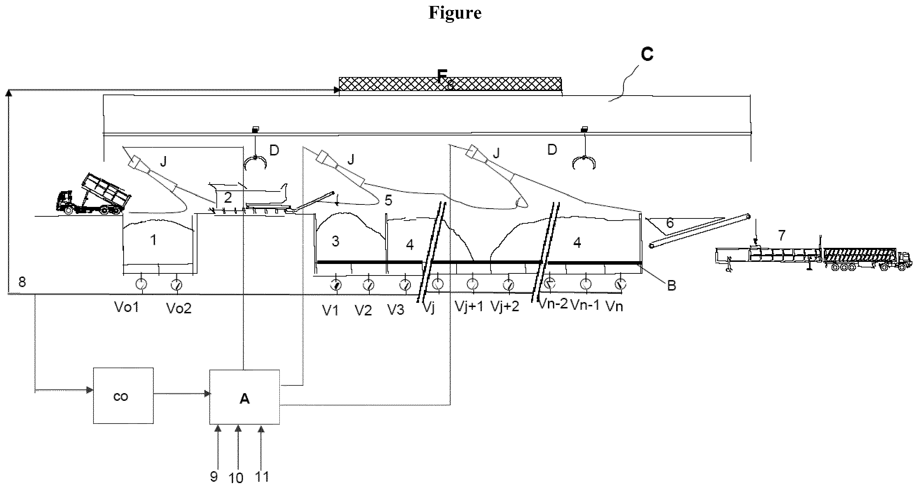

In the patent EP 1 386 675 A2 in the name of the same Applicant an automated system for the bio-drying of the waste and the relative control procedures are described in detail. Referring to the drawing, which shows the closed bio-drying chamber ***C,*** the waste is discharged in the reception pit 1, is shredded in 2 and discharged in 3 from where it is taken by the gantry crane buckets ***D*** and placed in **4** for digestion on a perforated floor ***B*** under forced aeration by the ventilators ***V***.

The perforated floor ***B*** is divided into sectors, each one served by a ventilator whose flow is regulated independently according to the type and the degree of digestion of the overlying waste.

Once the required state of bio-drying has been achieved and specifically a humidity content around 18% in weight, the material is taken from the buckets ***D*** and transferred 6 to a compactor 7 for the packing in bales or for transport.

Systems of this type are in operation in Italy and mainly in Lombardy and Piedmont but also in Europe and mainly the UK, Scotland, Spain and Greece.

The technology which is the object of the patent EP 1 386 675 A2 has moreover been applied for the composting of the wet fraction derived from the selective collection and of the biomasses of agricultural origin.

The same type of system is undergoing application to the storage of fuel derived from waste as a system of supply for incinerators.

It is therefore of industrial application importance to be able to guarantee in all cases the safety of these systems in respect of unexpected events which may be the origin or the cause of fires and the methods for containing them in the case wherein they occur.

Mention has already been made previously of the role of shredding for limiting local phenomena of anaerobiosis.

It is added here how the system of aspiration of air from above downwards through the heap of material stored is able to guarantee the containing of a possible fire.

It has been seen in fact, in simulated fires, that spherical nuclei of fire are formed, submerged in the material, having an extension of no more than 80- 90 cm in diameter, due to the fact that the flow of air downwards contrasts Archimedes' buoyancy force and prevents the extension of the fire to the whole mass.

However these submerged fires are often insidious and cannot be detected by the other sensors present on the system such as the smoke sensors present in the ceiling of the industrial building or by the temperature sensors on the intake of the ventilators and often remain ignored until the bio-dried mass is broken up or when, due to the de-actuation of a ventilator, they rise to the surface with disruptive effects.

Their origin may be the presence of biogas or of fine material which ignites due to the presence of electrostatic charges or more commonly the anomalous rising of the temperature in a mass of dry and fine waste in a zone bordering wet material undergoing digestion.

In fact the dry waste is normally stable while the wet waste is not reactive and normally digests, bringing its temperature to approximately 70°C but, if the temperature of the dry waste exceeds 70°C also locally, combustion may be triggered which propagates, giving rise to a pocket of fire in the mass.

This pocket may remain in the waste and self-extinguish or migrate into the mass due to instability of the flows until stopping on the walls of the industrial building and possibly making itself evident.

EP 1 258 280 A2 discloses a different composting and bio-drying waste facility than EP 1 386 675 A2, which is equipped with a system to detect possible submerged fires in a certain sector of the facility through measuring CO-content in the air by sucking air from different sectors and analysing the CO content of the sampled air. When the submerged fire has propagated it is often necessary to take drastic action with jets of water and with the breaking-up of the whole mass of waste with loss of production even of one week.

This disadvantage has imposed a redefinition of the methods and means for containing submerged fires and of the methods to be implemented in the case where their onset is to be avoided, with particular reference to stocks of reactive material such as that already treated and awaiting incineration.

### Disclosure of the invention

The general object of the present invention is that of eliminating the disadvantages stated above, making available a method and a system for the containing of fires which may occur in closed areas of storage and treatment of combustible solids such as for example systems for the composting of biomasses, systems of bio-drying of municipal solid waste, systems for the storage of waste and of refuse-derived fuels before incineration.

A further object is that of making available a method and a system for the containing of the fire that is fully automated and that is to say does not require the intervention of the operators and which allows regular production to be restarted after a reduced length of time from its actuation.

A particular object is that of making available a system and a method for creating permanent safety in areas of storage of solids which do not require special needs of treatment, such as for example refuse-derived fuels to be fed to the incinerators.

These and other objects which will be explained in greater detail herein below are achieved by a method and a system inserted in the system for bio-drying by means of forced digestion of waste containing putrescible fractions described in EP 1 386 675 A2, whereto reference will be made by way of nonlimiting description.

The drawing shows a longitudinal section of the system described in EP 1 386 675 A2 and wherein the elements useful for the present invention are shown.

The bio-drying system according to the prior art comprises a substantially closed digestion chamber ***C*** wherein waste is moved by means of the gantry crane and the buckets ***D*** which move longitudinally in the chamber, managed automatically by a process computer.

On the bottom of said chamber a support surface ***B*** is placed, divided longitudinally into sectors for the layered depositing of the waste, said support surface being provided with evenly distributed apertures for the passage through the layer of waste **1,3,4** of the air aspirated from above downwards by means of the ventilators ***V*** and sent via the line **8** to the bio-filters ***F*** before emission into the environment.

According to the invention and referring to the accompanying drawing, further apparatuses and sensors are inserted in said system:
- on the collector **8** of the air aspirated from all the sectors and which goes to the bio-filters ***F*** a sensor of carbon monoxide ***CO*** is placed, such as for example the FGA instrument by Land Instrument International, which has shown greater sensitivity for the monitoring of incipient submerged fires. It has in fact been noted that the submerged fire always takes place in the condition of partial combustion with production of considerable quantities of carbon monoxide. This sensor is integrated in the control software of the system, actuates the alarms and enables the procedure of containing of the fire.
- In the industrial building provision is made for the feeding of foams **5** produced by conventional systems constituted for example, as illustrated in the drawing, by a mixer ***A*** of water **9,** air or inert gases **10** and surfactants **11** and the jets ***J.*** The foam covers the surface of the layer of waste, suffocates the surface fires and is aspirated by the ventilators at depth.

For the purpose of implementation of the method that is the object of the present invention further information is necessary.

The surface speed of passage of the air or of the gas in the layer of waste is around 0.1 - 0.3 cm/s and the layer is about 5 m high, so that a phenomenon which occurs at the surface takes approximately half an hour to be noticed on the bottom.

The surface fires are easily monitored by the smoke/flame sensors or by equivalent apparatuses placed in the closed building and are detected with lag by the CO sensors placed on the air intake; they are rare even if more disruptive phenomena.

The submerged fires are more problematical since more contained and therefore less evident and can have various causes such as the presence in the waste of objects that release combustible vapours or gases or anaerobic pockets which produce biogas in the presence of electrostatic charges or phenomena of self-combustion of the dry matter or other sources of ignition of an incipient fire.

As already mentioned, suspended nuclei are formed in the mass of waste of dimensions contained to approximately 80 - 90 cm in equilibrium between the flow of air aspirated in the layer and the buoyancy force.

If the equilibrium were perfect they would extinguish by themselves having exhausted the material and suffocated by the combustion gases produced.

In reality variations in flow of the ventilators or lack of evenness of the motion mean that they can migrate while still submerged, generating other adjacent nuclei until arriving at the wall.

They are however detected more rapidly by the CO sensors alone.

Moreover surface fires are easily suffocated by feeding of foam in the sector or in the sectors where they occur and the operations planned and described herein below for the submerged fires are optional and cautionary.

Submerged fires on the contrary require a more complex procedure of longer duration for their interception and extinguishing and a possible final check by moving of the layer of material.

Therefore, according to the method that is the object of the present invention, as soon as the carbon monoxide sensor ***CO*** placed on the aspiration pipe **8** detects a concentration of carbon monoxide higher than 150 ppm and therefore the presence of a submerged fire, feeding of the foam is automatically actuated over the entire layer of waste by means of the mixer ***A*** and the jets ***J*** until covering the waste with a layer of foam of approximately 2 m.

At this point the ventilators ***V*** are automatically de-actuated, leaving the layer of waste without air supply and protected by the layer of foam for a period of at least 2 hours.

The ventilators ***V*** are actuated and de-actuated according to a preset sequence so as to aspirate the gas present in each individual sector and locate in which sector of the system the submerged fire is present by means of the ***CO*** detector.

Once the zone has been located action is taken with the bucket ***D,*** digging in the waste and, once the incipient fire has been found, extinguishing it with the water jets of the fire-fighting water supply system.

Once the waste has been broken up and when the content of carbon monoxide drops below 70 ppm, all the ventilators are re-actuated and normal production is restarted.

In any case the stoppage of production is always less than one day.

The consumption of foam and of the possible inert gases is limited if compared with the volumes of the industrial building constituting the closed area wherein the waste is accumulated, as will be indicated by the following examples.

### Detailed description of examples of embodiment

### EXAMPLE 1

A pilot experiment was performed on a container 3 m wide, 5 m long and 3 m high, filled with bio-dried material for approximately 2.5 m, subjected to aspiration of air from the bottom by means of a ventilator and equipped with a CO and temperature sensor on the aspiration pipe.

An incandescent electrical resistor was subsequently inserted, immersed in the waste at the depth of approximately 2 m, with the aim of causing a submerged fire.

Having actuated the resistor and under aspiration, presences of fire or of smoke at the surface were not noted while the CO sensor turned after a few minutes at 600 ppm.

Having injected as previously the compressed air foam, it was necessary to wait for about half an hour for the temperature and CO sensors to reach normal values.

Having removed the material some pockets of burnt material were noted, measuring from 80 to 30 cm.

The fire was in any case extinguished completely and signs of restarting during the operation of removal of the material were not seen.

### EXAMPLE 2

We shall consider an industrial building 20 m wide, 105 m long and 14 m high, wherein waste is accumulated in layers of approximately 6 m, divided along the length into 14 sectors of aspiration of the air by 14 ventilators below the support surface of the waste itself.

The volume of the industrial building is approximately 30000 m3 and that of the waste approximately 12000 m3 with a degree of vacuum around 0.6-0.7.

The total flow aspirated by the ventilators is 45000 m3/h and on average 3200 m3/h per sector.

In industrial buildings of this type 1-2 fires per year have occurred in the past out of a statistic of four systems operated.

The first was a submerged fire caused probably by a canister of camping gas and by electrostatic charges.

Presences of smoke at the surface were not seen until the ventilators randomly stopped.

The intervention with strong jets of water of the fire-fighting system installed entailed flooding of the plenum under the support surface of the waste of the sector involved, the saturation of the material stored with water and in the end the removal of all the material.

During this operation the presence of burnt pockets of material measuring 80-90 cm was noted, submerged at a level of 3 m from the bottom and which stopped at the wall.

The fire was put out yet it entailed the stop of the activity of the entire system for approximately five days with relative loss of material and of production.

The second fire occurred at the surface in a hopper for reception of the refined material, finely shredded and dry and therefore particularly reactive.

This was a surface fire triggered probably by a metallic part overheated due to friction by the shredder which fell onto a heap which had not yet been levelled.

The fire detected by the sensors installed was disruptive, filled the industrial building with smoke and damaged some wall structures.

In this case too the intervention with the water jets installed and with those of the firemen required several hours in order to be put out and entailed the same difficulties as the previous case.

Seeing the results of the pilot experiment of EXAMPLE 1 and given the delicacy of the operation, a test was performed with only the submerged fire in the pit of the bio-dried and shredded waste **3** and additionally all the operations provided by this invention for creating safety conditions of the entire industrial building were implemented manually.

Having buried an electrical resistor in the waste at approximately mid height in the layer of the pit **3** (3 m), the fire was ignited during the normal operations of the system and with the ventilators in operation.

After approximately ten minutes the CO sensors detected concentrations of approximately 70 ppm while no phenomenon was sensed at the surface of the waste.

There was a wait of a further ten minutes until the concentration of the CO reached 150 ppm and then proceeding with the following programme:
- covering of the whole system with a layer of foam 2 m thick, de-actuation of all the ventilators and wait of approximately 2 hours.
- Search for the submerged fire, re-actuating and de-actuating one by one all the ventilators and locating of the pit **3** as responsible for the fire.
- Breaking-up of the waste and prompt extinguishing of the fire.

The entire operation took approximately three hours from the start of injection of the foam.

The third fire occurred on a system similar to that described previously yet was activated spontaneously.

The system had already been equipped with all the automatic safety procedures.

In this case the detector of the CO rose rapidly up to 150 ppm while the spreading of the foam was actuated automatically until the entire layer of waste was covered and the ventilators were switched off.

The operator on duty restarted and switched off the ventilators one by one, moving from the two ends of the industrial building and specifically V1, V14, V2, V13 etc. for a time of 5 minutes per ventilator.

Sector 5 was identified as responsible for the fire with possible extensions to zones 4 and 6 with concentrations of CO up to 1200 ppm.

The empty zone where extraction of the already bio-dried material was underway was in 9.

Confirmation was therefore given for the mission of the gantry crane which moved onto sector 5 and started to dig systematically the entire zone from the centre to the walls and to return the material extracted into the empty operating zone.

After approximately 30 minutes an incipient fire surfaced at approximately 4 m from the wall and 3 m in depth.

The operators, already positioned with hydrants outside the industrial building on the walls bordering the sector, sprayed the zone for a radius of approximately 7 m.

Every sign of flames and smoke having disappeared, the ventilators V4, V5 and V6 were restarted and, having checked the return of the CO below the threshold of 20 ppm descending, all the other ventilators.

The process could therefore start again approximately 3 hours after detection.

The method that forms the object of the present invention therefore allows not only surface fires but also submerged ones, more insidious as less visible, to be put out in short times, avoiding losses of material and of production and damage to the structures of the building.

The quantity of water fed on the materials is limited to a few cubic metres and can easily be eliminated during the continuation of the operation of the system by means of the bio-drying process.

## Claims

1. System for locating and containing submerged fires in an aerated and closed chamber (C) for composting and bio-drying of waste and storage of solid flammable materials, said chamber (C) having on the base a support surface (B) divided longitudinally into sectors for the layered depositing of waste (1, 3, 4) by means of gantry crane buckets (D), said support surface being provided with evenly distributed apertures for the passage through the layer of waste (1, 3, 4) of the air aspirated from above downwards by means of ventilators (V) and sent via a line (8) with bio-filters (F) before emission into the environment, wherein said system comprises:
- a sensor (S) of carbon monoxide placed on said aspiration line (8), designed to monitor the concentration of CO in said chamber (C);
- jets (J) for feeding foams (5) on the surface of the layer of waste (1, 3, 4) when said sensor (S) detects a concentration of CO higher than a preset threshold;
- means suitable for actuating and de-actuating in sequence the ventilators (V) of the various sectors of the chamber (C) so as to aspirate the gas present in each individual sector of the chamber (C) and locate in which sector the submerged fire is present on the basis of the concentration of CO detected.

2. System according to claim 1, wherein said sensor (S) can be the FGA instrument of Land Instrument International.

3. System according to claim 1, wherein said foams (5) are fed to the jets (J) by a mixer (A) of water (9), air or inert gases (10) and surfactants (11).

4. Method for locating and containing submerged fires in an aerated and closed chamber (C) for composting and bio-drying of waste and storage of solid flammable materials, wherein said chamber (C) has on the base a perforated support surface (B) divided longitudinally into sectors for the layered depositing of waste (1, 3, 4), and wherein provision is made for the aspiration of air from above downwards through the layer of waste (1, 3, 4) by means of ventilators (V) and the treatment of the aspirated air before emission into the environment, wherein said method comprises the following phases:
- monitoring of the concentration of CO in said chamber (C);
- feeding of foam on the surface of the layer of waste (1, 3, 4) when a concentration of CO higher than a first preset threshold is detected;
- de-actuation of all the ventilators (V) leaving the layer of waste without air supply and protected by the layer of foam;
- actuation and de-actuation of the ventilators (V) according to a preset sequence so as to aspirate the gas present in each individual sector of the chamber (C) and locate in which sector the submerged fire is present on the basis of the concentration of CO detected;
- monitoring of the concentration of CO in the sector located, keeping the relative ventilators (V) actuated in order to check whether the concentration of CO exceeds a second preset threshold, higher than the first, in which case digging is carried out in the sector located and the source of the fire extinguished with water jets, or whether the concentration of CO drops below said first threshold, in which case all the ventilators (V) are actuated, restarting normal production.

5. Method according to claim 4, wherein said first threshold of concentration of CO is 70 ppm.

6. Method according to claim 5, wherein said second threshold of concentration of CO is 150 ppm.

7. Method according to claim 4, wherein all the ventilators (V) are de-actuated leaving the layer of waste without air supply for approximately 15 minutes.

8. Method according to claim 4, wherein said foam is obtained by mixing water, air or inert gases and surfactants and is fed to jets (J) for feeding on the layer of waste.

## Patentansprüche

1. System zum Lokalisieren und Eindämmen von unter der Oberfläche liegenden Bränden in einer belüfteten und geschlossenen Kammer (C) zur Kompostierung und Bio-Trocknung von Müll und Lagerung von festen brennbaren Materialien, wobei die Kammer (C) auf der Grundseite eine Auflagefläche (B) aufweist, die längs in Sektoren für die schichtförmige Abscheidung von Müll (1, 3, 4) mittels Brückenkranschaufeln (D) unterteilt ist, wobei die Auflagefläche mit gleichmäßig verteilten Öffnungen für den Durchlass der Luft durch die Müllschicht (1, 3, 4) versehen ist, die von oben nach unten mittels Ventilatoren (V) angesaugt und vor der Emission in die Umwelt durch eine Leitung (8) mit Biofiltern (F) geleitet wird, wobei
das System umfasst:
- einen Kohlenmonoxid-Sensor (S), der auf der Ansaugleitung (8) angeordnet und dazu bestimmt ist, die CO-Konzentration in der Kammer (C) zu überwachen;
- Düsen (J) zur Zuführung von Schäumen (5) auf die Oberfläche der Müllschicht (1, 3, 4), wenn der Sensor (S) eine CO-Konzentration erfasst, die höher als ein voreingestellter Schwellenwert ist;
- Mittel, die zur aufeinander folgenden Aktivierung und Deaktivierung der Ventilatoren (V) der verschiedenen Sektoren der Kammer (C) geeignet sind, um das in jedem einzelnen Sektor der Kammer (C) vorhandene Gas anzusaugen und auf der Grundlage der erfassten CO-Konzentration zu lokalisieren, in welchem Sektor der unter der Oberfläche liegende Brand vorhanden ist.

2. System nach Anspruch 1, wobei der Sensor (S) der FGA-Apparat von Land Instruments International sein kann.

3. System nach Anspruch 1, wobei die Schäume (5) den Düsen (J) durch einen Mischer (A) für Wasser (9), Luft oder Inertgase (10) und Tenside (11) zugeführt werden.

4. Verfahren zum Lokalisieren und Eindämmen von unter der Oberfläche liegenden Bränden in einer belüfteten und geschlossenen Kammer (C) zur Kompostierung und Bio-Trocknung von Müll und Lagerung von festen brennbaren Materialien, wobei die Kammer (C) auf der Grundseite eine gelochte Auflagefläche (B) aufweist, die längs in Sektoren für die schichtförmige Abscheidung von Müll (1, 3, 4) unterteilt ist und wobei die Ansaugung von Luft durch die Müllschicht (1, 3, 4) von oben nach unten mittels Ventilatoren (V) und die Behandlung der angesaugten Luft vor der Emission in die Umwelt vorgesehen ist, wobei
das Verfahren folgende Phasen umfasst:
- Überwachen der CO-Konzentration in der Kammer (C);
- Zuführen von Schaum auf die Oberfläche der Müllschicht (1, 3, 4), wenn eine CO-Konzentration erfasst wird, die höher als ein voreingestellter Schwellenwert ist;
- Deaktivieren aller Ventilatoren (V), so dass die Müllschicht ohne Luftzufuhr belassen wird und durch die Schaumschicht geschützt bleibt;
- Aktivieren und Deaktivieren der Ventilatoren (V) in einer voreingestellten Folge, um das in jedem einzelnen Sektor der Kammer (C) vorhandene Gas anzusaugen und auf der Grundlage der erfassten CO-Konzentration zu lokalisieren, in welchem Sektor der unter der Oberfläche liegende Brand vorhanden ist;
- Überwachen der CO-Konzentration in dem lokalisierten Sektor, wobei die entsprechenden Ventilatoren (V) aktiviert bleiben, um zu prüfen, ob die CO-Konzentration einen zweiten voreingestellten Schwellenwert überschreitet, der höher als der erste ist,
in welchem Fall das Ausheben in dem lokalisierten Sektor vorgenommen und die Brandquelle mit Wasserstrahlen gelöscht wird, oder ob die CO-Konzentration unter den ersten Schwellenwert sinkt, in welchem Fall alle Ventilatoren (V) aktiviert werden und die normale Produktion wieder aufgenommen wird.

5. Verfahren nach Anspruch 4, wobei der erste Schwellenwert der CO-Konzentration bei 70 ppm liegt.

6. Verfahren nach Anspruch 5, wobei der zweite Schwellenwert der CO-Konzentration bei 150 ppm liegt.

7. Verfahren nach Anspruch 4, wobei alle Ventilatoren (V) deaktiviert werden, so dass die Müllschicht ungefähr 15 Minuten ohne Luftzufuhr belassen wird.

8. Verfahren nach Anspruch 4, wobei der Schaum durch Mischen von Wasser, Luft oder Inertgasen und Tensiden erhalten und den Düsen (J) zum Zuführen auf die Müllschicht zugeführt wird.

## Revendications

1. Système de localisation et de confinement des incendies submergés dans une chambre aérée et fermée (C) pour compostage et bio-séchage de déchets et stockage de matériaux inflammables solides, ladite chambre (C) ayant sur la base une surface de support (B) divisée longitudinalement en secteurs pour le dépôt en couches de déchets (1, 3, 4) au moyen de godets de grue à portique (D), ladite surface de support étant pourvue d'ouvertures régulièrement réparties pour le passage à travers la couche de déchets (1, 3, 4) de l'air aspiré de haut en bas au moyen de ventilateurs (V) et envoyé par une ligne (8) avec des biofiltres (F) avant d'être émis dans l'environnement, dans lequel
ledit système comprend:
- un capteur (S) de monoxyde de carbone placé sur ladite ligne d'aspiration (8), destiné à surveiller la concentration de CO dans ladite chambre (C);
- des jets (J) pour amener des mousses (5) sur la surface de la couche de déchets (1, 3, 4) lorsque ledit capteur (S) détecte une concentration de CO supérieure à un seuil prédéterminé;
- des moyens aptes à actionner et à désactiver en séquence les ventilateurs (V) des différents secteurs de la chambre (C) de manière à aspirer le gaz présent dans chaque secteur individuel de la chambre (C) et localiser dans quel secteur l'incendie submergé est présent sur la base de la concentration de CO relevée.

2. Système selon la revendication 1, dans lequel ledit capteur (S) peut être l'instrument FGA de Land Instrument International.

3. Système selon la revendication 1, dans lequel lesdites mousses (5) sont amenées aux jets (J) par un mélangeur (A) d'eau (9), d'air ou de gaz inertes (10) et d'agents tensioactifs (11).

4. Procédé de localisation et de confinement des incendies submergés dans une chambre aérée et fermée (C) pour compostage et bio-séchage de déchets et stockage de matériaux inflammables solides, dans lequel ladite chambre (C) a sur la base une surface de support perforée (B) divisée longitudinalement en secteurs pour le dépôt par couches de déchets (1, 3, 4), et dans lequel on prévoit l'aspiration de l'air de haut en bas à travers la couche de déchets (1, 3, 4) au moyen de ventilateurs (V) et du traitement de l'air aspiré avant d'être émis dans l'environnement, dans lequel ledit procédé comprend les phases suivantes:
- surveiller la concentration de CO dans ladite chambre (C);
- amener la mousse à la surface de la couche de déchets (1, 3, 4) lorsqu'une concentration de CO supérieure à un premier seuil prédéfini est relevé;
- désactiver tous les ventilateurs (V) quittant la couche de déchets sans apport d'air et protégée par la couche de mousse;
- actionner et désactiver les ventilateurs (V) selon une séquence prédéfinie de façon à aspirer le gaz présent dans chaque secteur individuel de la chambre (C) et localiser dans quel secteur l'incendie submergé est présent sur la base de la concentration de CO relevée;
- contrôler la concentration de CO dans le secteur localisé, en gardant les ventilateurs relatifs (V) actionnés pour vérifier si la concentration de CO dépasse un second seuil prédéterminé, supérieur au premier,
dans ce cas, le creusement est effectué dans le secteur localisé et la source de l'incendie est éteinte avec des jets d'eau, ou si la concentration de CO tombe en dessous dudit premier seuil, auquel cas tous les ventilateurs (V) sont actionnés, relançant la production normale.

5. Procédé selon la revendication 4, dans lequel ledit premier seuil de concentration de CO est de 70 ppm.

6. Procédé selon la revendication 5, dans lequel ledit deuxième seuil de concentration de CO est de 150 ppm.

7. Procédé selon la revendication 4, dans lequel tous les ventilateurs (V) sont désactivés, laissant la couche de déchets sans apport d'air pendant environ 15 minutes.

8. Procédé selon la revendication 4, dans lequel ladite mousse est obtenue en mélangeant de l'eau, de l'air ou des gaz inertes et des agents tensioactifs et est amenée à des jets (J) pour l'alimentation de la couche de déchets.
